# EUROPEAN PATENT SPECIFICATION

(11) **EP 4 509 035 B1**
(45) Date of publication and mention of the grant of the patent: **11.03.2026**
(21) Application number: 24193948.7
(22) Date of filing: 09.08.2024
(51) Int. Cl.: A61B 3/107, F21S 2/00, F21V 3/02

(54) **PLACIDO DISK LIGHTING DEVICE AND METHOD FOR MANUFACTURING THE SAME**
PLACIDOSCHEIBENBELEUCHTUNGSVORRICHTUNG UND VERFAHREN ZUR HERSTELLUNG DAVON
DISPOSITIF D'ÉCLAIRAGE À DISQUE PLACIDO ET SON PROCÉDÉ DE FABRICATION

(30) Priority: 14.08.2023 KR 20230106544
(43) Date of publication of application: 19.02.2025
(73) Proprietor: Huvitz Co., Ltd., Anyang-si, Gyeonggi-do 14055 (KR)
(72) Inventor: PARK, Sang Jeon, 14055 Anyang-si (KR); LIM, Jong Min, 14055 Anyang-si (KR); KIM, Nam Woon, 14055 Anyang-si (KR)
(74) Representative: IPAZ

(56) References cited:
- EP-B1- 2 925 208
- CN-A- 108 420 399

## Description

### FIELD OF THE DISCLOSURE

The present invention relates to a Placido disk lighting device and a method of manufacturing the same, and more specifically, to a Placido disk lighting device that can be easily manufactured by mounting a flexible light-emitting diode circuit board on which a plurality of light-emitting diodes (LEDs) are mounted on the inner surface of a hemispherical housing and a method for manufacturing the same.

### BACKGROUND

A corneal shape examination device is a device that examines the surface shape of a cornea of an eye and is a device that radiates the cornea with light of a predetermined shape and analyzes the degree to which the image (shape) of the light formed on the cornea is deformed, thereby examining the curvature of each part of the cornea. This system is also commonly referred to as a corneal curvature meter (corneal topographer or keratoscope). This corneal shape examination device is mainly used to examine changes in the shape of the cornea before and after irregular astigmatism, keratoconus, and refractive surgeries. Corneal shape examination devices are classified into a Placido disk method and a slit scan method, and the present invention relates to the Placido disk method.

Early corneal curvature meters measure the curvature of the cornea by injecting a single ring-shaped light with an infrared wavelength into the cornea, detecting the image of the ring reflected from the cornea with a camera, and then analyzing its shape. However, in this case, only the curvature of the central region of the cornea can be known. Therefore, currently, a corneal shape examination device is being used that can measure even the peripheral area of the cornea by increasing the number of rings, that is, by irradiating the cornea with light having the shape of a plurality of rings arranged concentrically. In this corneal shape examination device, a Placido disk lighting device is used to irradiate the cornea with light having the shape of a plurality of rings arranged concentrically.

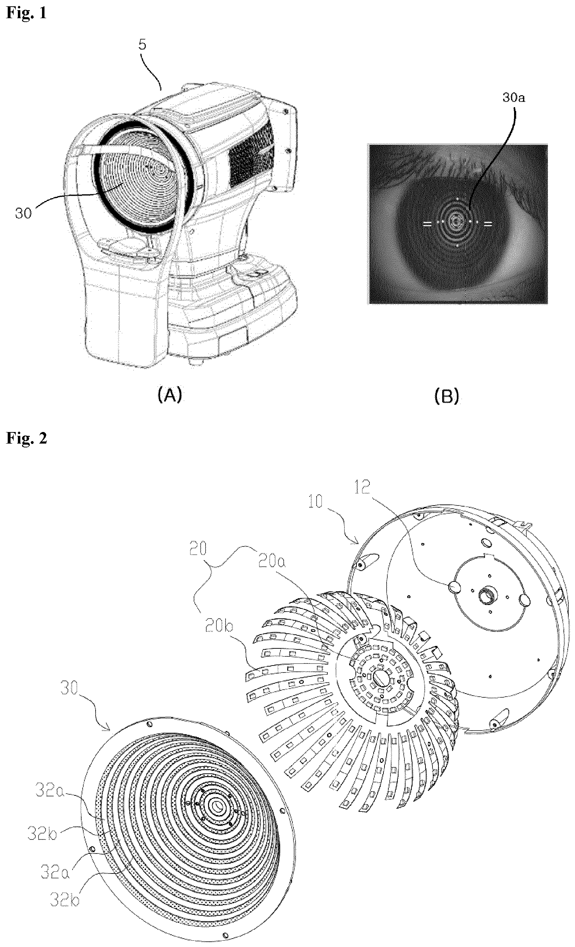

FIG. 1A is a diagram showing a corneal shape examination device including a conventional Placido disk lighting device and FIG. 1B is a diagram showing a Placido ring pattern being formed on the cornea of a subject eye using the same. As shown in FIG. 1A, a Placido disk 30 for irradiating the cornea with light having the shape of a plurality of rings arranged concentrically is mounted on the front surface of a conventional corneal shape examination device 5. When the subject eye is placed in front of the Placido disk 30, as shown in FIG. 1B, an image 30a of the Placido disk 30 having the shape of a plurality of rings is formed on the corneal surface of the subject eye.

In the Placido disk method shown in FIGS. 1A and 1B, when the cornea is irradiated with light having the shape of a plurality of rings arranged concentrically from the Placido disk 30, a camera attached to the center position of the Placido disk 30 obtains the ring-shaped image 30a reflected from the cornea. In this way, the image of each ring can be detected, the curvature value at each position of the cornea can be calculated from the image, and the corneal surface shape can be calculated.

Here, the Placido disk 30 must be illuminated so that the image 30a of the Placido ring-shaped measurement light obtained by the camera is clearly and uniformly visible. If the brightness of the Placido disk lighting is not uniform, errors may occur in the analysis of the image 30a obtained with the camera.

Generally, a direct lighting method, an indirect lighting method, or a combination of the direct lighting method and the indirect lighting method are used to illuminate the Placido disk 30.

In the direct lighting method, a measurement light source, for example, a plurality of light-emitting diodes (LEDs), is placed on the back surface of the Placido disk 30 which has the Placido ring shape. This direct lighting method has difficulties in manufacturing because each LED used as a measurement light source must be fixed at a predetermined angle according to the shape of the Placido disk 30.

In the indirect lighting method, a measurement light source is located on the front surface of the Placido disk 30 and uses light reflected from the Placido disk 30. This indirect lighting method has a problem in that the brightness of the light reflected from the Placido disk 30 cannot be adjusted locally.

Therefore, there is a demand for a Placido disk lighting device which is easy to mass produce and in which the brightness of a measurement light source can be adjusted locally so that the image of Placido ring-shaped measurement light is uniformly visible, and there is no need to individually adjust the mounting angles of the measurement light sources, that is, a plurality of LEDs.

### Citation List

### Patent Literature

Document 1: Korean Patent Application Publication No. 10-2008-0067040. EP2925208B1. CN108420399A.
EP 2 925 208 B1 discloses a Placido disk projector for corneal topography with flexible printed circuit board (1) having radial cuts (11) creating strips (14), applied to paraboloid support (3) using adhesive. LEDs (15) are distributed in annular arrays. The board is shaped to match support convexity (paragraphs [0012]-[0018], [0020]). Circuit board is "close to spherical"

CN 108420399 A discloses a bowl-shaped support frame (3) with aluminum-based LED circuit board (2) having central circular base and radially extending branches (10). LEDs (11) mounted in ring distributions. The branches are fixed to the support via bolts (paragraphs [0006]-[0008], [0027]-[0036]).

### SUMMARY OF THE DISCLOSURE

### TECHNICAL OBJECTS

An object of the present invention is to provide a Placido disk lighting device that can be easily manufactured by mounting a flexible light-emitting diode circuit board on which a plurality of light-emitting diodes (LEDs) is mounted on the inner surface of a hemispherical housing, and a method for manufacturing the same.

Another object of the present invention is to provide a Placido disk lighting device and a method for manufacturing the same capable of adjusting the illumination brightness of a Placido disk used in a corneal shape examination device for each position.

### TECHNICAL SOLUTION

In order to achieve the above object, the present invention provides a Placido disk lighting device including: a hemispherical housing having a hemispherical shape; a light-emitting diode circuit board including a central circuit board mounted on an inner surface of the hemispherical housing and a plurality of flexible radial circuit boards extending radially from the central circuit board, in which a plurality of light-emitting devices are mounted at predetermined intervals on the central circuit board and the radial circuit boards ; and a Placido disk mounted on a front surface of the light-emitting diode circuit board and having a shape of a plurality of concentric Placido rings, the Placido disk emitting light having a shape of a plurality of concentric Placido rings when the light-emitting device mounted on the light-emitting diode circuit board emits light.

The present invention also provides a method for manufacturing a Placido disk lighting device, the method including: mounting a light-emitting diode circuit board on an inner surface of a hemispherical housing having a hemispherical shape, the light-emitting diode circuit board including a central circuit board and a plurality of flexible radial circuit boards extending radially from the central circuit board, in which a plurality of light-emitting devices are mounted at predetermined intervals on the central circuit board and the radial circuit boards ; and mounting a Placido disk on a front surface of the hemispherical housing on which the light-emitting diode circuit board is mounted and the Placido disk having a shape of a plurality of concentric Placido rings, the Placido disk emitting light having a shape of a plurality of concentric Placido rings when the light-emitting device mounted on the light-emitting diode circuit board emits light.

### EFFECTS OF THE DISCLOSURE

According to the Placido disk lighting device and the method for manufacturing the same according to the present invention, the Placido disk lighting device can be easily manufactured by mounting the flexible light-emitting diode circuit board on which a plurality of light-emitting diodes is mounted on the inner surface of a hemispherical housing.

### BRIEF DESCRIPTION OF THE DRAWINGS

FIGS. 1A and 1B are diagrams showing a corneal shape examination device including a conventional Placido disk lighting device and a Placido ring pattern being formed on the cornea of a subject eye using the same.
FIG. 2 is an exploded perspective view of a Placido disk lighting device according to an embodiment of the present invention.
FIG. 3A is a diagram showing a light-emitting diode circuit board used in a Placido disk lighting device according to an embodiment of the present invention and FIG. 3B is a diagram showing a fixing member for fixing the light-emitting diode circuit board to a hemispherical housing.
FIG. 4 is a photograph showing a state in which a light-emitting diode circuit board is mounted on a hemispherical housing in the Placido disk lighting device according to an embodiment of the present invention.

### DETAILED DESCRIPTION OF THE PREFERRED EMBODIMENTS

Hereinafter, the present invention will be described in detail with reference to the attached drawings. In the description of the present invention, detailed descriptions of related well-known general-purpose structures or functions will be omitted.

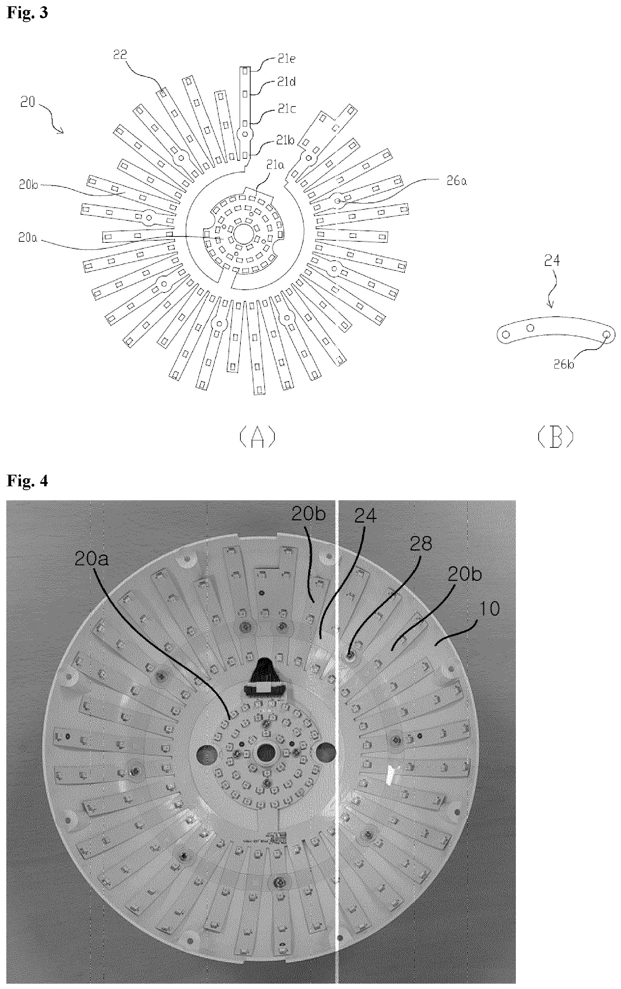

FIG. 2 is an exploded perspective view of a Placido disk lighting device according to an embodiment of the present invention, FIG. 3A is a diagram showing a light-emitting diode circuit board used in a Placido disk lighting device according to an embodiment of the present invention and FIG. 3B is a diagram showing a fixing member for fixing a light-emitting diode circuit board 20 to a hemispherical housing 10. As shown in FIG. 2 and FIGS. 3A and 3B, the Placido disk lighting device according to the present invention includes: a hemispherical housing 10 having a hemispherical shape; a light-emitting diode circuit board 20 including a central circuit board 20a mounted on an inner surface of the hemispherical housing 10 and a plurality of flexible radial circuit boards 20b extending radially from the central circuit board 20a, in which a plurality of light-emitting devices 22, advantageously light-emitting diodes (LED) are mounted at predetermined intervals on the central circuit board 20a and the radial circuit boards 20b; and a Placido disk 30 mounted on a front surface of the light-emitting diode circuit board 20 and having the shape of a plurality of concentric Placido rings, the Placido disk 30 emitting light having a shape of a plurality of concentric Placido rings (that is, light having a shape of a plurality of rings arranged concentrically) when the light-emitting device 22 mounted on the light-emitting diode circuit board 20 emits light.

As shown in FIG. 2, the hemispherical housing 10 is a housing having a hemispherical shape, and plays the role of shaping the Placido disk lighting device into a hemispherical shape (structure), and is also called a back cap.

The hemispherical housing 10 is coupled with the light-emitting diode circuit board 20, the Placido disk 30, and the like, which will be described later, and serves to support them.

A hole 12 may be formed in the hemispherical housing 10 so as to pass typical optometry light, for example, aiming light for fixing the gaze of the subject eye and focusing light for adjusting the distance between a corneal shape measurement device and the subject eye.

As shown in FIG. 3A, the light-emitting diode circuit board 20 includes a central circuit board 20a and a plurality of flexible radial circuit boards 20b extending radially from the central circuit board 20a.

The light-emitting diode circuit board 20 is preferably a flexible board with flexibility made up of a single circuit structure, and for example, may be a flexible printed circuit board (PCB).

On the central circuit board 20a and the radial circuit board 20b of the light-emitting diode circuit board 20, a plurality of light sources 22 are uniformly mounted at predetermined intervals to irradiate the Placido disk 30 with light so that the cornea of the subject eye is irradiated with Placido ring-shaped light (that is, light in the shape of a plurality of rings arranged concentrically). The light source 22 is a measurement light source for irradiating the subject eye with Placido ring-shaped measurement light and may be the light-emitting device 22 (LED), specifically, the light-emitting device 22 (LED) that emits infrared measurement light rather than visible light.

The light-emitting diodes 22 mounted on the light-emitting diode circuit board 20 are provided (disposed) so that the ring shape of the Placido disk 30 is formed uniformly over the entire cornea. The light-emitting diodes 22 are preferably disposed at the same distance from the center. The light-emitting diodes 22 are preferably radially and uniformly disposed on the light-emitting diode circuit board 20, specifically on a plurality of flexible radial circuit boards 20b.

FIG. 4 is a photograph showing a state in which the light-emitting diode circuit board 20 is mounted on the hemispherical housing 10 in the Placido disk lighting device according to an embodiment of the present invention. As shown in FIGS. 3A and 3B and FIG. 4, the light-emitting diode circuit board 20 has a flat shape before being combined with the hemispherical housing 10, but is bent at a predetermined angle while being fixed to the hemispherical housing 10, forming a three-dimensional hemispherical shape. Specifically, since the circuit board 20 includes the central circuit board 20a and a plurality of flexible radial circuit boards 20b extending radially from the central circuit board 20a, the circuit board 20 can be fixed to the hemispherical housing 10 in a curved state. Here, the central circuit board 20a is also preferably flexible. Therefore, the plurality of light-emitting diodes 22 mounted on the light-emitting diode circuit board 20 are fixed to the hemispherical housing 10 designed with a constant curvature without additional angle adjustment, the light emitted from the light-emitting diodes 22 proceeds at a predetermined angle, and the Placido disk 30 is uniformly irradiated with a uniform amount of light.

Preferably, the Placido disk lighting device according to the present invention may further include a fixing member 24 for pressing and fixing one end of the flexible radial circuit board 20b to the hemispherical housing 10. The fixing member 24 serves as a fixing bar for fixing the light-emitting diode circuit board 20 to the hemispherical housing 10. As shown in FIG. 3B, the fixing member 24 preferably has an arc shape with a predetermined curvature. The fixing member 24 has a curved shape such as an arc, and can fix the flexible radial circuit board 20b along the curve of the hemispherical housing 10.

The light-emitting diode circuit board 20 may be fixed (connected) using a plurality of fixing members 24 instead of one fixing member 24. The material of the fixing member 24 may be a material capable of pressing and fixing the circuit board 20b to the hemispherical housing 10, for example, flexible plastic. Preferably, a predetermined fixing hole 26b is formed in the fixing member 24. It is preferable that one to three fixing holes 26b are formed in the fixing member 24.

The fixing member 24 can fix (connect) the light-emitting diode circuit board 20 to the hemispherical housing 10 using conventional coupling means such as screws. As shown in FIG. 4, the hemispherical housing 10, the radial circuit board 20b, and the fixing member 24 may be fixed by coupling means 28 such as screws. A fixing hole 26a, which is fixed to the hemispherical housing 10 by the coupling means 28, may be formed at one end of the flexible radial circuit board 20b. The fixing hole 26b of the fixing member 24 and the fixing hole 26a formed at one end of the radial circuit board 20b are fixed and coupled by the coupling means 28. The coupling means 28 may be any means for fixing the hemispherical housing 10, the flexible radial circuit board 20b, and the fixing member 24.

In an embodiment of the present invention, the light-emitting diodes 22 mounted on the light-emitting diode circuit board 20 may form a plurality of channels, for example, two to fifteen channels, preferably three to ten channels, more preferably four to seven channels, for example, five channels (21a, 21b, 21c, 21d, and 21e) as shown in FIGS. 3A and 3B. Specifically, the light-emitting diode 22 is mounted on the central circuit board 20a to form one first light-emitting diode channel 21a, for example. In addition, the light-emitting diodes 22 are mounted on the radial circuit board 20b in the shape of a plurality of rings arranged concentrically around the central circuit board 20a, thereby forming a plurality of second, third, fourth, and fifth light-emitting diode channels 21b, 21c, 21d and 21e. Here, the light-emitting diodes 22 located at the same distance from the central circuit board 20a form the same channel. That is, a plurality of light-emitting diodes 22 are arranged on the radial circuit board 20b sequentially from the central circuit board 20a to form a plurality of light-emitting diode channels 21b, 21c, 21d, and 21e. For example, as shown in FIGS. 3A and 3B, four light-emitting diode channels 21b, 21c, 21d, and 21e are formed on the radial circuit board 20b along four rings arranged concentrically. At this time, the light-emitting diodes 22 mounted on respective rings form respective channels.

A brightness of the light-emitting diode circuit board 20 is adjustable for each of the light-emitting diode channels 21a, 21b, 21c, 21d, and 21e. The light-emitting diodes 22 mounted on the light-emitting diode circuit board 20 form a plurality of light-emitting diode channels 21a, 21b, 21c, 21d, and 21e, so that the brightness of the light-emitting devices 22 can be adjusted for each of the light-emitting diode channels 21a, 21b, 21c, 21d, and 21e. The light-emitting diodes 22 preferably form channels in the same shape as the ring pattern of the Placido disk 30. In the light-emitting diode circuit board 20, since the brightness of the light-emitting diodes 22 can be adjusted for each of the channels 21a, 21b, 21c, 21d, and 21e, the lighting brightness of the entire Placido disk 30 can be adjusted uniformly. When the ring lighting of a specific portion (channel) needs to be adjusted arbitrarily, the brightness of the light-emitting device 22 of desired channel(s) can be adjusted partially (locally). Therefore, the lighting brightness of the Placido disk ring can be adjusted as needed.

In the Placido disk lighting device according to the present invention, by using a single flexible light-emitting diode circuit board 20, there is no need to separately adjust the mounting angle of the light-emitting diode 22 mounted on the circuit board 20. In the Placido disk lighting device according to the present invention, the flexible light-emitting diode circuit board 20 is fixed to the hemispherical housing 10 having a predetermined curvature, and the light-emitting diode (22) emits light at a predetermined angle according to the curvature of the hemispherical housing 10.

In the Placido disk lighting device according to the present invention, the measurement light source, that is, the light-emitting diode 22, is not directly fixed to the hemispherical housing 10, but a single light-emitting diode circuit board 20 in which the light-emitting diodes 22 are mounted at predetermined intervals is fixed to the hemispherical housing 10. Additionally, the brightness of the light-emitting diodes 22 can be uniformly or locally adjusted according to the channels 21a, 21b, 21c, 21d, and 21e of the light-emitting diode circuit board 20.

The Placido disk 30 is provided with a plurality of concentric Placido rings spaced at predetermined intervals so as to emit light in the shape of a plurality of concentric Placido rings (that is light in the shape of a plurality of rings arranged concentrically). Specifically, as shown in FIG. 2, the Placido disk 30 includes a plurality of ring-shaped measurement light transmitting portions 32a arranged concentrically to transmit the measurement light emitted from the light-emitting diode 22; and a measurement light blocking portion 32b located between the measurement light transmitting portions 32a to block transmission of the measurement light.

That is, the Placido disk 30 has the plurality of ring-shaped measurement light transmitting portions 32a that transmits the measurement light and the measurement light blocking portions 32b that block the measurement light, which are alternately formed to generate light having the shape of a plurality of concentric Placido rings. Accordingly, when the light-emitting diode 22 radiates measurement light from the circuit board 20, the Placido disk 30 emits light in the shape of a plurality of concentric Placido rings (that is, light in the shape of a plurality of rings arranged concentrically).

Light in the shape of a plurality of concentric Placido rings emitted from the Placido disk lighting device according to the present invention is reflected from the cornea of the subject eye and is detected by a camera (not shown) of the corneal shape examination device 5 (see FIGS. 1A and 1B) and used for detecting the shape of the cornea.

In the present invention, the light-emitting diode 22 is mounted at a desired angle while the light-emitting diode circuit board 20 is fixed to the hemispherical housing 10 designed with a predetermined curvature, and one light-emitting diode circuit board 20 is used. Therefore, mass production is easy.

In a corneal shape examination device, the mounting direction of the light-emitting diode is important to ensure uniform brightness of the entire Placido disc. In the past, the light-emitting diodes had to be mounted at predetermined angles, which was disadvantageous in manufacturing.

On the other hand, the Placido disk lighting device according to the present invention does not need to adjust the mounting angle of each of the light-emitting diodes 22. In addition, since the light-emitting diode circuit board 20 includes the central circuit board 20a and the flexible radial circuit board 20b, the Placido disk lighting device can be controlled using one integrated light-emitting diode circuit board 20.

Next, the method for manufacturing the Placido disk lighting device according to the present invention will be described. According to the present invention, in order to manufacture the Placido disk lighting device, first, the light-emitting diode circuit board 20 is mounted on the inner surface of the hemispherical housing 10 having a hemispherical shape, the light-emitting diode circuit board 20 including the central circuit board 20a and the plurality of flexible radial circuit boards 20b extending radially from the central circuit board 20a, in which a plurality of light-emitting devices 22 are mounted at predetermined intervals on the central circuit board 20a and the radial circuit boards 20b. At this time, preferably, one end of the flexible radial circuit board 20b is pressed and fixed to the hemispherical housing 10 using the fixing member 24. Next, the Placido disk 30 is mounted on the front surface of the hemispherical housing 10 on which the light-emitting diode circuit board 20 is mounted, the Placido disk 30 having the shape of a plurality of concentric Placido rings, the Placido disk 30 emitting light having the shape of a plurality of concentric Placido rings when the light-emitting device 22 mounted on the light-emitting diode circuit board 20 emits light. The Placido disk 30 is mounted on the front surface of the hemispherical housing 10 to which the light-emitting diode circuit board 20 is fixed.

The light-emitting diode circuit board 20 may be located on the inner surface of the hemispherical housing 10, specifically in a concave portion with a predetermined curvature, and one end of the flexible radial circuit board 20b may be pressed and fixed to the hemispherical housing 10 using the fixing member 24. The number of fixing members 24 used may vary depending on the size of the hemispherical housing 10.

In the Placido disk lighting device according to the present invention, the light-emitting diode circuit board 20 on which a plurality of light-emitting diodes 22 are mounted is fixed and coupled to the hemispherical housing 10 using the fixing member 24, and the front surface of the light-emitting diode circuit board 20 is fixed and mounted on the front surface of the hemispherical housing 10 to form the Placido disk lighting device. Therefore, the Placido disk lighting device can be manufactured simply and easily.

In addition, the intensity of light emitted from the light-emitting diodes 22 can be uniformly adjusted for each of the channels 21a, 21b, 21c, 21d, and 21e of the light-emitting diode circuit board 20, or the light intensity can be adjusted locally only in a desired portion. Therefore, it is possible to more easily irradiate the cornea with the light having the shape of a plurality of rings arranged concentrically.

Although the present invention has been described above with reference to the accompanying drawings and exemplary embodiments, the present invention is not limited to the content shown in the drawings and the above-described embodiments. Although reference numerals are used in the following claims to aid understanding, the scope of the following claims is not limited to the reference numerals and the content shown in the drawings, and should be interpreted to encompass all variations of exemplary embodiments within the scope of the claims.

## Claims

1. A Placido disk lighting device comprising:
a hemispherical housing (10) having a hemispherical shape;
a light-emitting diode circuit board (20) including a central circuit board (20a) mounted on an inner surface of the hemispherical housing (10) and a plurality of flexible radial circuit boards (20b) extending radially from the central circuit board (20a), in which a plurality of light-emitting devices (22) are mounted at predetermined intervals on the central circuit board (20a) and the radial circuit boards (20b);
a fixing member (24) having a curved shape, for pressing and fixing one end of the flexible radial circuit board (20b) to the hemispherical housing (10); and
a Placido disk (30) mounted on a front surface of the light-emitting diode circuit board (20) and having a shape of a plurality of concentric Placido rings, the Placido disk (30) emitting light having a shape of a plurality of concentric Placido rings when the light-emitting device (22) mounted on the light-emitting diode circuit board (20) emits light,
wherein the light-emitting diode circuit board (20) has a flat shape before being combined with the hemispherical housing (10), but is bent at a predetermined angle while being fixed to the hemispherical housing (10), forming a three-dimensional hemispherical shape.

2. The Placido disk lighting device according to claim 1, wherein
the light-emitting diode (22) is mounted on the central circuit board (20a) to form one light-emitting diode channel (21a), and
the light-emitting devices (22) are attached to the radial circuit board (20b) in a shape of a plurality of rings arranged concentrically around the central circuit board (20a) to form a plurality of light-emitting diode channels (21b, 21c, 21d, 21e).

3. The Placido disk lighting device according to claim 2, wherein a brightness of the light-emitting diode circuit board (20) is adjustable for each of the light-emitting diode channels (21a, 21b, 21c, 21d, and 21e).

4. The Placido disk lighting device according to claim 1, wherein the Placido disk (30) includes:
a plurality of ring-shaped measurement light transmitting portions (32a) arranged concentrically to transmit the measurement light emitted from the light-emitting diode (22); and
a measurement light blocking portion (32b) located between the measurement light transmitting portions (32a) to block transmission of the measurement light.

5. A method for manufacturing a Placido disk lighting device, the method comprising:
mounting a light-emitting diode circuit board (20) on an inner surface of a hemispherical housing (10) having a hemispherical shape, the light-emitting diode circuit board (20) including a central circuit board (20a) and a plurality of flexible radial circuit boards (20b) extending radially from the central circuit board (20a), in which a plurality of light-emitting devices (22) are mounted at predetermined intervals on the central circuit board (20a) and the radial circuit boards (20b);
pressing and fixing one end of the flexible radial circuit board (20b) to the hemispherical housing (10) with a fixing member (24) having a curved shape; and
mounting a Placido disk (30) on a front surface of the hemispherical housing (10) on which the light-emitting diode circuit board (20) is mounted, the Placido disk (30) having a shape of a plurality of concentric Placido rings, the Placido disk (30) emitting light having a shape of a plurality of concentric Placido rings when the light-emitting device (22) mounted on the light-emitting diode circuit board (20) emits light,
wherein the light-emitting diode circuit board (20) has a flat shape before being combined with the hemispherical housing (10), but is bent at a predetermined angle while being fixed to the hemispherical housing (10), forming a three-dimensional hemispherical shape.

## Patentansprüche

1. Placido-Scheibenbeleuchtungsvorrichtung, umfassend:
ein halbkugelförmiges Gehäuse (10) mit einer halbkugelförmigen Gestalt;
eine Leuchtdioden-Leiterplatte (20), einschließend eine zentrale Leiterplatte (20a), die an einer Innenfläche des halbkugelförmigen Gehäuses (10) montiert ist, und eine Vielzahl von flexiblen radialen Leiterplatten (20b), die sich radial von der zentralen Leiterplatte (20a) erstrecken, wobei eine Vielzahl von Leuchtvorrichtungen (22) in vorbestimmten Abständen auf der zentralen Leiterplatte (20a) und den radialen Leiterplatten (20b) montiert sind;
ein Befestigungselement (24) mit gekrümmter Form zum Andrücken und Befestigen eines Endes der flexiblen radialen Leiterplatte (20b) an dem halbkugelförmigen Gehäuse (10); und
eine Placido-Scheibe (30), die auf einer Vorderseite der Leuchtdioden-Leiterplatte (20) montiert ist und die Form einer Vielzahl konzentrischer Placido-Ringe aufweist, wobei die Placido-Scheibe (30) Licht in Form einer Vielzahl von konzentrischen Placido-Ringen emittiert, wenn die auf der Leuchtdioden-Leiterplatte (20) montierte Leuchtvorrichtung (22) Licht emittiert,
wobei die Leuchtdioden-Leiterplatte (20) vor dem Verbinden mit dem halbkugelförmigen Gehäuse (10) eine flache Form aufweist, jedoch beim Befestigen an dem halbkugelförmigen Gehäuse (10) in einem vorbestimmten Winkel gebogen wird, wodurch eine dreidimensionale halbkugelförmige Form entsteht.

2. Placido-Scheibenbeleuchtungsvorrichtung nach Anspruch 1, wobei
die Leuchtdiode (22) auf der zentralen Leiterplatte (20a) montiert ist, um einen Leuchtdiodenkanal (21a) zu bilden, und
die Leuchtvorrichtungen (22) an der radialen Leiterplatte (20b) in Form einer Vielzahl von Ringen montiert sind, die konzentrisch um die zentrale Leiterplatte (20a) angeordnet sind, um eine Vielzahl von Leuchtdiodenkanälen (21b, 21c, 21d, 21e) zu bilden.

3. Placido-Scheibenbeleuchtungsvorrichtung nach Anspruch 2, wobei die Helligkeit der Leuchtdioden-Leiterplatte (20) für jeden der Leuchtdiodenkanäle (21a, 21b, 21c, 21d und 21e) einstellbar ist.

4. Placido-Scheibenbeleuchtungsvorrichtung nach Anspruch 1, wobei die Placido-Scheibe (30) Folgendes einschließt:
eine Vielzahl von ringförmigen Messlichtübertragungsabschnitten (32a), die konzentrisch angeordnet sind, um das von der Leuchtdiode (22) emittierte Messlicht zu übertragen; und
ein Messlichtblockierabschnitt (32b), der sich zwischen den Messlichtübertragungsabschnitten (32a) befindet, um die Übertragung des Messlichts zu blockieren.

5. Verfahren zum Herstellen einer Placido-Scheibenbeleuchtungsvorrichtung, das Verfahren umfassend:
Montieren einer Leuchtdioden-Leiterplatte (20) an einer Innenfläche eines halbkugelförmigen Gehäuses (10) mit einer halbkugelförmigen Gestalt, wobei die Leuchtdioden-Leiterplatte (20) eine zentrale Leiterplatte (20a) und eine Vielzahl von flexiblen radialen Leiterplatten (20b) einschließt, die sich radial von der zentralen Leiterplatte (20a) erstrecken, wobei eine Vielzahl von Leuchtvorrichtungen (22) in vorbestimmten Abständen auf der zentralen Leiterplatte (20a) und den radialen Leiterplatten (20b) montiert sind;
Andrücken und Befestigen eines Endes der flexiblen radialen Leiterplatte (20b) an dem halbkugelförmigen Gehäuse (10) mit einem Befestigungselement (24), das eine gekrümmte Form aufweist; und
Montieren einer Placido-Scheibe (30) auf einer Vorderseite des halbkugelförmigen Gehäuses (10), auf dem die Leuchtdioden-Leiterplatte (20) montiert ist, wobei die Placido-Scheibe (30) die Form einer Vielzahl von konzentrischen Placido-Ringen aufweist, wobei die Placido-Scheibe (30) Licht in Form einer Vielzahl von konzentrischen Placido-Ringen emittiert, wenn die auf der Leuchtdioden-Leiterplatte (20) montierte Leuchtvorrichtung (22) Licht emittiert,
wobei die Leuchtdioden-Leiterplatte (20) vor dem Verbinden mit dem halbkugelförmigen Gehäuse (10) eine flache Form aufweist, jedoch beim Befestigen an dem halbkugelförmigen Gehäuse (10) in einem vorbestimmten Winkel gebogen wird, wodurch eine dreidimensionale halbkugelförmige Form entsteht.

## Revendications

1. Dispositif d'éclairage à disque de Placido comprenant :
un boîtier hémisphérique (10) ayant une forme hémisphérique ;
une carte de circuit imprimé à diodes électroluminescentes (20) comprenant une carte de circuit imprimé centrale (20a) montée sur une surface intérieure du boîtier hémisphérique (10) et une pluralité de cartes de circuit imprimé radiales flexibles (20b) s'étendant radialement depuis la carte de circuit imprimé centrale (20a), dans laquelle une pluralité de dispositifs électroluminescents (22) sont montés à des intervalles prédéterminés sur la carte de circuit imprimé centrale (20a) et les cartes de circuit imprimé radiales (20b) ;
un élément de fixation (24) ayant une forme incurvée, pour presser et fixer une extrémité de la carte de circuit imprimé radiale flexible (20b) au boîtier hémisphérique (10) ; et
un disque de Placido (30) monté sur une surface avant de la carte de circuit imprimé à diodes électroluminescentes (20) et ayant une forme d'une pluralité d'anneaux de Placido concentriques, le disque de Placido (30) émettant une lumière ayant une forme d'une pluralité d'anneaux de Placido concentriques lorsque le dispositif électroluminescent (22) monté sur la carte de circuit imprimé à diodes électroluminescentes (20) émet de la lumière,
dans lequel la carte de circuit imprimé à diodes électroluminescentes (20) a une forme plane avant d'être assemblée avec le boîtier hémisphérique (10), mais est pliée selon un angle prédéterminé lors de sa fixation au boîtier hémisphérique (10), formant une forme hémisphérique tridimensionnelle.

2. Dispositif d'éclairage à disque de Placido selon la revendication 1, dans lequel
la diode électroluminescente (22) est montée sur la carte de circuit imprimé centrale (20a) pour former un canal de diodes électroluminescentes (21a), et
les dispositifs électroluminescents (22) sont fixés à la carte de circuit imprimé radiale (20b) sous une forme d'une pluralité d'anneaux agencés de manière concentrique autour de la carte de circuit imprimé centrale (20a) pour former une pluralité de canaux de diodes électroluminescentes (21b, 21c, 21d, 21e).

3. Dispositif d'éclairage à disque de Placido selon la revendication 2, dans lequel une luminosité de la carte de circuit imprimé à diodes électroluminescentes (20) est réglable pour chacun des canaux de diodes électroluminescentes (21a, 21b, 21c, 21d et 21e).

4. Dispositif d'éclairage à disque de Placido selon la revendication 1, dans lequel le disque de Placido (30) comprend :
une pluralité de parties de transmission de lumière de mesure (32a) de forme annulaire agencées de manière concentrique pour transmettre la lumière de mesure émise depuis la diode électroluminescente (22) ; et
une partie de blocage de lumière de mesure (32b) située entre les parties de transmission de lumière de mesure (32a) pour bloquer la transmission de la lumière de mesure.

5. Procédé de fabrication d'un dispositif d'éclairage à disque de Placido, le procédé comprenant :
le montage d'une carte de circuit imprimé à diodes électroluminescentes (20) sur une surface intérieure d'un boîtier hémisphérique (10) ayant une forme hémisphérique, la carte de circuit imprimé à diodes électroluminescentes (20) comprenant une carte de circuit imprimé centrale (20a) et une pluralité de cartes de circuit imprimé radiales flexibles (20b) s'étendant radialement depuis la carte de circuit imprimé centrale (20a), dans laquelle une pluralité de dispositifs électroluminescents (22) sont montés à des intervalles prédéterminés sur la carte de circuit imprimé centrale (20a) et les cartes de circuit imprimé radiales (20b) ;
le pressage et la fixation d'une extrémité de la carte de circuit imprimé radiale flexible (20b) au boîtier hémisphérique (10) avec un élément de fixation (24) ayant une forme incurvée ; et
le montage d'un disque de Placido (30) sur une surface avant du boîtier hémisphérique (10) sur lequel la carte de circuit imprimé à diodes électroluminescentes (20) est montée, le disque de Placido (30) ayant une forme d'une pluralité d'anneaux de Placido concentriques, le disque de Placido (30) émettant une lumière ayant une forme d'une pluralité d'anneaux de Placido concentriques lorsque le dispositif électroluminescent (22) monté sur la carte de circuit imprimé à diodes électroluminescentes (20) émet de la lumière,
dans lequel la carte de circuit imprimé à diodes électroluminescentes (20) a une forme plane avant d'être assemblée avec le boîtier hémisphérique (10), mais est pliée selon un angle prédéterminé lors de sa fixation au boîtier hémisphérique (10), formant une forme hémisphérique tridimensionnelle.
